# EUROPÄISCHE PATENTANMELDUNG

(11) **EP 1 719 468 A1**
(43) Veröffentlichungstag der Anmeldung: **08.11.2006**
(21) Anmeldenummer: 04030000.6
(22) Anmeldetag: 17.12.2004
(51) Int. Cl.: A61B 17/70

(54) **Intervertebrales Stabilisierungssystem**

(71) Anmelder: Zimmer GmbH, 8404 Winterthur (CH)
(72) Erfinder: Egli, Thomas, 8305 Dietlikon (CH); Zylber, Emmanuel, 13008 Marseille (FR); Lancial, Michael Eugene, Minnesota 55426 (US)
(74) Vertreter: Manitz, Finsterwald & Partner GbR

(57) **Zusammenfassung**

Intervertebrales Stabilisierungssystem für wenigstens drei Wirbel, mit
- an den Wirbeln befestigbaren Pedikelschrauben (67),
- wenigstens einem Stab (11) zum Verbinden von zumindest zwei Pedikelschrauben zu einem starren Versteifungssystem, und
- zumindest einem auf Zug vorspannbaren Band (23), das im implantierten Zustand des Stabilisierungssystems von wenigstens einem zwischen zwei benachbarten Pedikelschrauben angeordneten komprimierbaren Druckkörper (77) umgeben ist, zum Verbinden der Pedikelschrauben zu einem elastischen Stützsystem,
wobei eine gemeinsame Pedikelschraube sowohl dem Versteifungssystem als auch dem Stützsystem zugeordnet und das Band mittels einer Bandbefestigung mit dem Stab verbindbar oder verbunden ist.

## Beschreibung

Die Erfindung betrifft ein intervertebrales Stabilisierungssystem für wenigstens drei Wirbel.

Bei Deformationen oder degenerativen Veränderungen der Wirbelsäule kann es nötig sein, das jeweils betroffene instabile Wirbelsäulensegment operativ zu stabilisieren.

Hierfür ist es bekannt, das betroffene Wirbelsäulensegment zu versteifen. Bei einer derartigen partiellen Wirbelsäulenversteifung werden üblicherweise die Bandscheiben zumindest teilweise entfernt, Knochengewebe zwischen die zu versteifenden Wirbel eingelagert und die zu versteifenden Wirbel mittels Schrauben und zumindest eines Stabes starr miteinander verbunden. Die Versteifung des Wirbelsäulensegments bedingt jedoch, dass die benachbarten Wirbel bei Beuge- und Streckbewegungen der Wirbelsäule stärker als vor der Versteifung beansprucht werden.

Alternativ kommt neuerdings "bei leichteren Fällen", insbesondere noch bevor ein Bandscheibenvorfall auftritt, ein Implantat zum Einsatz, das das betroffene Wirbelsäulensegment von posterior stützt, aber nicht versteift und so die Beweglichkeit der betroffenen Wirbel weitestgehend erhält. Dabei wird zwischen an den Wirbeln befestigten Schrauben ein elastisches Band mit biegsamen Abstandshaltern eingesetzt. Das elastische Band begrenzt die Beugebewegung, während die Abstandshalter die Streckbewegung limitieren.

Der Erfindung liegt die Aufgabe zugrunde, eine Vorrichtung der eingangs genannten Art zu schaffen, die dazu ausgelegt ist, ein instabiles Wirbelsäulensegment derart zu versteifen, dass bei Beuge- und Streckbewegungen die Wirbelsäule möglichst wenig beansprucht wird, insbesondere um weitere Operationen zu verhindern oder zumindest weitmöglichst hinauszuzögern.

Die Lösung dieser Aufgabe erfolgt durch die Merkmale des Anspruchs 1 und insbesondere dadurch, dass an den Wirbeln befestigbare Pedikelschrauben, ein Stab zum Verbinden von zumindest zwei Pedikelschrauben zu einem starren Versteifungssystem und ein auf Zug vorspannbares Band, das im implantierten Zustand des Stabilisierungssystems von wenigstens einem zwischen zwei benachbarten Pedikelschrauben angeordneten komprimierbaren Druckkörpern umgeben ist, zum Verbinden der Pedikelschrauben zu einem elastischen Stützsystem vorgesehen sind, wobei eine gemeinsame Pedikelschraube sowohl dem Versteifungssystem als auch dem Stützsystem zugeordnet und das Band mittels einer Bandbefestigung mit dem Stab verbindbar oder verbunden ist.

Die Erfindung zeichnet sich folglich dadurch aus, dass das aus dem Stand der Technik bekannte starre Versteifungssystem, das den Stab umfasst, mit dem ebenfalls aus dem Stand der Technik bekannten elastischen Stützsystem, das das Band und den Druckkörper umfasst, kombiniert wird. Das bislang alternativ zu dem bekannten starren Versteifungssystem verwendete elastische Stützsystem wird erfindungsgemäß nun gemeinsam mit dem starren Versteifungssystem zur Stabilisierung eines instabilen Wirbelsäulensegments eingesetzt.

Der Stab des Versteifungssystems ist zur Versteifung eines zumindest zwei Wirbel umfassenden instabilen Wirbelsäulensegments ausgelegt. Das Band, das im implantierten Zustand des Stabilisierungssystems mit dem Stab verbunden und im nicht implantierten Zustand mit dem Stab verbunden oder verbindbar ist, wird zur Stützung zumindest eines an das versteifte Wirbelsäulensegment angrenzenden Wirbels verwendet. Der Stab und das Band sind mittels der Pedikelschrauben an den zu stabilisierenden Wirbeln befestigt.

Vorzugsweise sind zumindest zwei Versteifungssysteme, insbesondere zwei Stäbe, vorgesehen, die im implantierten Zustand des Stabilisierungssystems links und rechts der Dornfortsätze der betroffenen Wirbel angeordnet sind, wobei bevorzugt die beiden Versteifungssysteme miteinander verbindbar oder verbunden sind. Ferner können mehrere Stützsysteme vorgesehen sein. Insbesondere können für das, für mehrere oder für jedes Versteifungssystem zwei Stützsysteme vorgesehen sein, die jeweils an ein Ende des Versteifungssystems anschließen. Grundsätzlich können auch mehrere Versteifungssysteme und Stützsysteme vorgesehen sein, die im implantierten Zustand alternierend miteinander verbunden sind.

Das Band des Stützsystems ist im implantierten Zustand des Stabilisierungssystems auf Zug vorgespannt. Der Druckkörper hingegen befindet sich in einem komprimierten Zustand, d.h. er ist auf Druck vorgespannt. Der Stab und das Band sind dabei über eine Bandbefestigung miteinander verbunden, d.h. das Band ist an dem Stab befestigt oder umgekehrt. Die gemeinsame Pedikelschraube, an der oder in deren Nähe im implantierten Zustand des Stabilisierungssystems die Verbindung des Bands mit dem Stab üblicherweise ausgebildet ist, wird von dem starren Versteifungssystem und dem elastischen Stützsystem gleichermaßen verwendet. Ist die Bandbefestigung etwa mittig zwischen zwei Pedikelschrauben angeordnet, kann eine beliebige der beiden Pedikelschrauben als die gemeinsame Pedikelschraube betrachtet werden.

Durch das elastische Stützsystem, das sich als Verlängerung des starren Versteifungssystems an dieses anschließt, bzw. durch die elastischen Stützsysteme, die sich an beiden Seiten des starren Versteifungssystems an dieses anschließen, können der oder die an die durch das Versteifungssystem starr miteinander verbundenen Wirbel anschließenden Wirbel gestützt werden, so dass diese benachbarten Wirbel bei Beuge- und Streckbewegungen der Wirbelsäule keinen erhöhten Belastungen ausgesetzt sind.

Vorteilhafte Ausführungsformen der Erfindung sind auch in den Unteransprüchen, der Beschreibung sowie der Zeichnung angegeben.

Vorzugsweise ist die Bandbefestigung als Klemmeinrichtung zur Klemmung des Bands ausgebildet. Durch die Klemmkräfte der als Klemmeinrichtung ausgelegten Bandbefestigung kann das im implantierten Zustand des Stabilisierungssystems unter Zugkraft stehende Band besonders sicher und zuverlässig gehalten werden.

Es ist weiterhin bevorzugt, dass die Bandbefestigung ein Ende des Stabs, eine separate Baueinheit und/oder die gemeinsame Pedikelschraube umfasst.

Die Erfindung wird im Folgenden beispielhaft unter Bezugnahme auf die Zeichnung, die verschiedene Ausführungsformen umfasst, deren jeweiligen Merkmale - soweit sinnvoll - beliebig miteinander kombinierbar sind, beschrieben. Es zeigen:
- Fig. 1 bis 7: Bandbefestigungen eines erfindungsgemäßen Stabilisierungssystems, die jeweils ein Ende des Stabs umfassen,
- Fig. 8 bis 10: Bandbefestigungen eines erfindungsgemäßen Stabilisierungssystems, die jeweils eine separate Baueinheit umfassen, und
- Fig. 11 bis 15: Bandbefestigungen eines erfindungsgemäßen Stabilisierungssystems, die jeweils eine gemeinsame Pedikelschraube umfassen.

Die nachstehend erläuterten Ausführungsformen zeigen jeweils nur den Teil des Stabilisierungssystems, der für die Beschreibung der Verbindung zwischen dem Stab des Versteifungssystems und dem Band des Stützsystems notwendig ist. Dabei sind gleiche oder einander entsprechende Bestandteile der verschiedenen Ausführungsformen der Erfindung jeweils mit denselben Bezugszeichen bezeichnet.

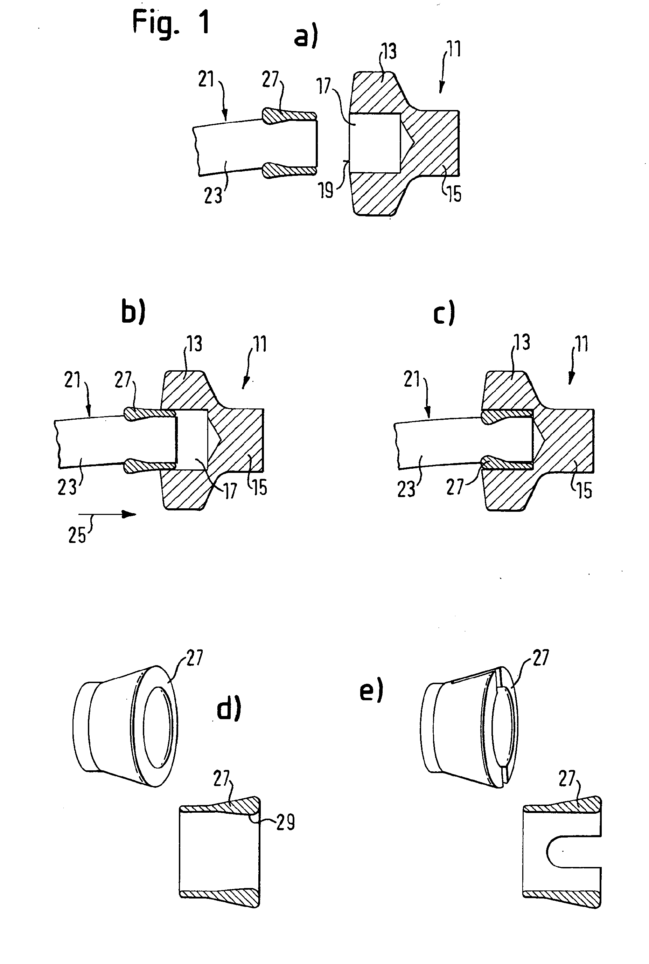

Fig. 1 zeigt ein Stabilisierungssystem gemäß einer ersten Ausführungsform der Erfindung mit einem aus Metall gefertigten starren Stab 11 eines ansonsten nicht dargestellten starren Versteifungssystems, wobei der Stab 11 ein Ende 13 aufweist, das einen größeren Durchmesser besitzt als das Mittelteil 15 des Stabs 11. Das als Kopf 13 ausgebildete Ende des Stabs 11, das gleichzeitig als Anschlag für einen nicht dargestellten Druckkörper dienen kann, ist zumindest Teil einer Bandbefestigung und weist einen im Wesentlichen zylinderartigen Hohlraum 17 auf, in den über eine Bandeintrittsöffnung 19 ein Ende 21 eines elastischen, beispielsweise aus Kunststoff gefertigten Bands 23 eines ansonsten nicht dargestellten elastischen Stützsystems einführbar oder eingeführt ist.

Die Bandbefestigung umfasst ferner ein in den Hohlraum 17 längs der Einführrichtung 25 des Endes 21 des Bands 23 einführbares (Fig. 1a) oder eingeführtes (Fig. 1c), in Umfangrichtung geschlossenes hülsenartiges Klemmelement 27, das auf das Ende 21 des Bands 23 aufgeschoben ist und zur Klemmung des in den Hohlraum 17 des Stabs 11 eingeführten Endes 21 des Bands 23 mit dem Kopf 13 des Stabs 11 ausgelegt ist. Das hülsenartige Klemmelement 27 kann auch als Klemmhülse oder Spannring bezeichnet werden.

Die Klemmhülse 27 ist im nicht in den Hohlraum 17 des Stabs 11 eingeführten Zustand an ihrer Außenseite konisch zulaufend ausgebildet, wobei die Wandstärke der Klemmhülse 27 in Richtung des den größeren Außendurchmesser aufweisenden Endes zunimmt. Gemäß Fig. 1d kann an der Innenseite der Klemmhülse 27 an dem den größeren Außendurchmesser aufweisenden Ende ein in Umfangsrichtung umlaufender, nach innen gerichteter, den Innendurchmesser der Klemmhülse 27 verengender Vorsprung 29 ausgebildet sein. Gemäß Fig. 1e kann das Klemmelement 27 insbesondere auf zwei gegenüberliegenden Seiten geschlitzt sein. Grundsätzlich kann die Klemmhülse sowohl einen Vorsprung aufweisen als auch geschlitzt sein.

In den Fig. 1a bis 1c ist das Verbinden des Stabs 11 mit dem Band 23 gezeigt. Zunächst wird die Klemmhülse 27 auf das in den Hohlraum 17 des Stabs 11 einzuführende Ende 21 des Bands 23 derart aufgeschoben, dass das den größeren Außendurchmesser aufweisende Ende der konischen Klemmhülse 27 der Mitte des Bands 23 zugewandt ist (Fig. 1a). Danach wird das mit der Klemmhülse 25 versehene Ende 21 des Bands 23 mittels eines geeigneten Werkzeugs längs der Einführrichtung 25 in den Hohlraum 17 des Kopfes 13 des Stabs 11 eingepresst (Fig. 1b).

Dabei wird das den größeren Außendurchmesser aufweisende Ende der Klemmhülse 27 auf den Innendurchmesser des zylinderartigen Hohlraums 17 des Stabs 11, der zumindest im Wesentlichen dem kleineren Außendurchmesser des anderen Endes der Klemmhülse 27 entspricht, zusammengedrückt. Im zusammengesetzten Zustand (Fig. 1c) nimmt der Innendurchmesser der Klemmhülse 27 in Richtung des ursprünglich einen größeren Außendurchmesser aufweisenden Endes aufgrund der in dieser Richtung zunehmenden Wandstärke der Klemmhülse 27 ab, d.h. der Durchgang der Klemmhülse 27 verengt sich in Richtung der Bandeintrittsöffnung 19 des Hohlraums 17 des Kopfes 13 des Stabs 11, so dass das Band 23 wirksam geklemmt wird.

Durch den in Fig. 1d dargestellten Vorsprung 29 kann die Klemmkraft der Bandbefestigung zusätzlich verstärkt werden. Eine geschlitzte Klemmhülse 27 (Fig. 1e) bietet den Vorteil, dass beim Einführen der Klemmhülse 27 in den Hohlraum 17 des Kopfes 13 des Stabs 11 zusätzlicher Platz für Materialverformungen zur Verfügung steht, so dass der Einpressvorgang insgesamt mit geringerem Kraftaufwand durchgeführt werden kann.

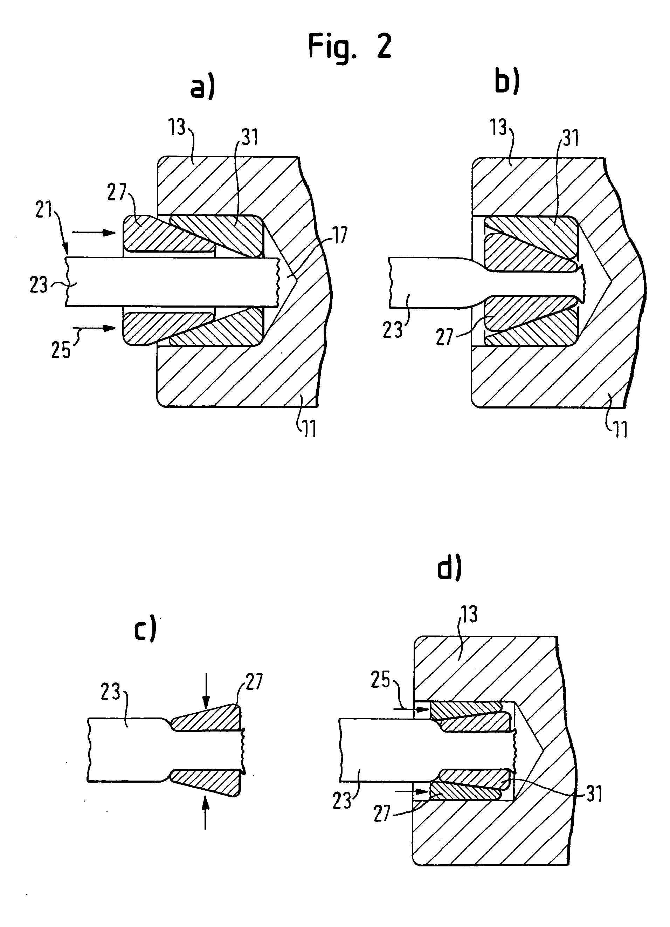

Fig. 2 zeigt ein Stabilisierungssystem gemäß einer zweiten Ausführungsform der Erfindung. Im Gegensatz zu der Ausführungsform in Fig. 1 umfasst die Bandbefestigung gemäß Fig. 2 zwei Klemmhülsen 27, 31, die gegeneinander verspannt sind.

In Analogie zu Fig. 1 ist bei dem Ausführungsbeispiel gemäß den Fig. 2a und 2b die erste Klemmhülse 27 auf das in den Hohlraum 17 des Stabs 11 einzuführende Ende 21 des Bands 23 derart aufgeschoben, dass das den größeren Außendurchmesser aufweisende Ende der konischen ersten Klemmhülse 27 der Mitte des Bands 23 zugewandt ist (Fig. 2a). Die zweite Klemmhülse 31, die als Kegelpressring dient, ist spiegelbildlich zu der ersten Klemmhülse 27 bezüglich einer Ebene, die senkrecht auf der Einführrichtung 25 steht, in den Hohlraum 17 des Kopfes 13 des Stabs 11 eingepresst (Fig. 2a). Zum Verbinden des Bands 23 mit dem Stab 11 wird die erste Klemmhülse 27 längs der Einführrichtung 25 in den Hohlraum 17 des Kopfes 13 des Stabs 11 eingepresst, wobei die erste Klemmhülse 27 mit der zweiten Klemmhülse 31 verspannt. Die erste Klemmhülse 27 wird dabei derart verformt, dass das Band 23 wirksam eingeklemmt ist (Fig. 2b). Grundsätzlich können auch mehrere Kegelpressringe vorgesehen sein.

Bei dem Ausführungsbeispiel gemäß den Fig. 2c und 2d sind die beiden Klemmhülsen 27, 31 hinsichtlich ihrer Orientierungen gegenüber dem Ausführungsbeispiel gemäß den Fig. 2a und 2b vertauscht, wobei die erste Klemmhülse 27 im nicht eingeführten Zustand auf das Ende 21 des Bands 23 aufgepresst ist (Fig. 2c). Beim Zusammensetzen wird das mit der ersten Klemmhülse 27 versehene Ende 21 des Bands 23 längs der Einführrichtung 25 in den Hohlraum 17 des Kopfes 13 des Stabs 11 eingeführt. Anschließend wird die zweite Klemmhülse 31 längs der Einführrichtung 25 in den Hohlraum eingepresst (Fig. 2d).

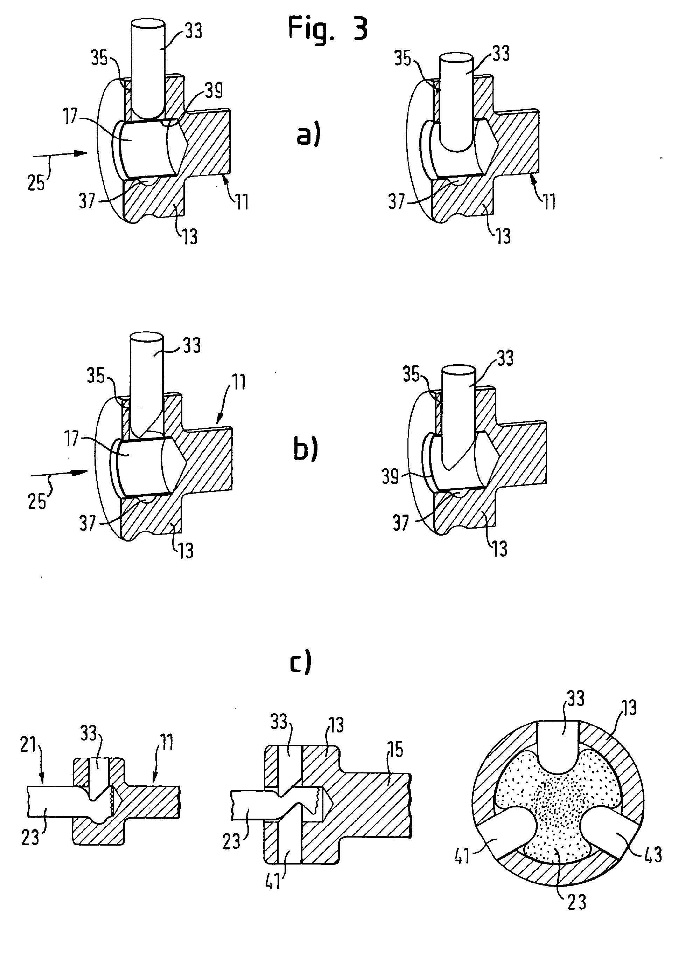

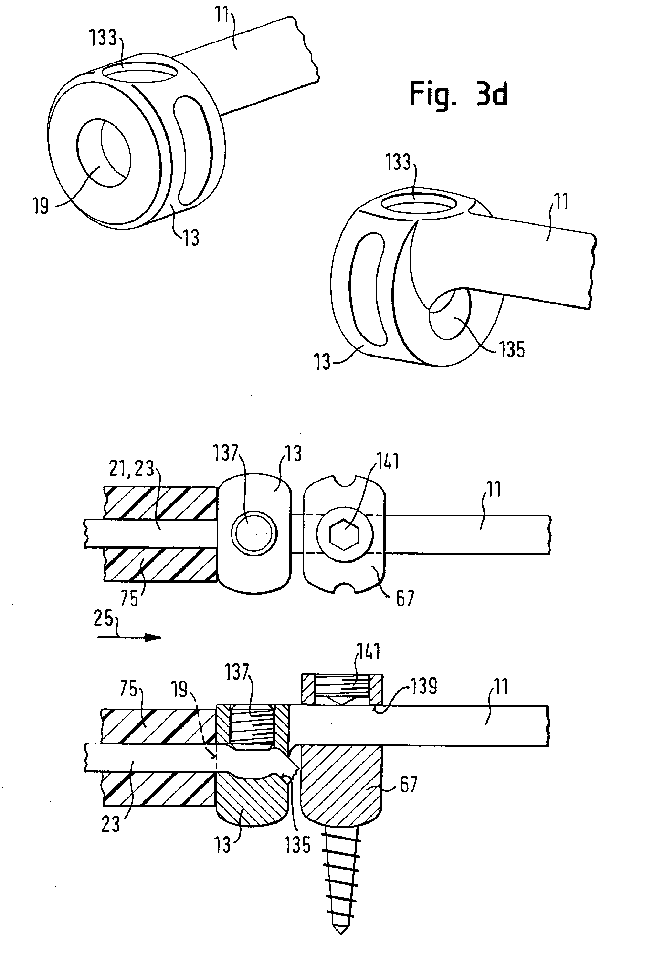

Fig. 3 zeigt ein Stabilisierungssystem gemäß einer dritten Ausführungsform der Erfindung.

Dabei umfasst die Bandbefestigung des Ausführungsbeispiels gemäß den Fig. 3a bis 3c einen unter Press-Sitz in den Hohlraum 17 quer zur Einführrichtung 25 des Endes 21 des Bands 23 einführbaren Klemmstift 33, der zur Klemmung des in den Hohlraum 17 des Stabs 11 eingeführten Endes des Bands mit dem Kopf 13 des Stabs 11 ausgelegt ist. Der Klemmstift 33, der durch eine Öffnung 35 in den Hohlraum 17 des Stabs 11 eingeführt ist, kann an seiner Spitze, die das eingeführte Ende 21 des Bands 23 radial zu dessen Längserstreckung mit Druck beaufschlagt, rund (Fig. 3a) oder keilförmig (Fig. 3b) ausgebildet sein. Mit einem keilförmigen Klemmstift 33 kann ein größerer Pressdruck auf das eingeklemmte Band 23 erreicht werden.

Diametral entgegengesetzt zu der Öffnung 35, durch die der Klemmstift 33 in den Hohlraum 17 eingeführt ist, ist an der den Hohlraum 17 begrenzenden Innenseite des Kopfes 13 des Stabs 11 eine Mulde 37 ausgebildet, in die das Band 23 bei eingepresstem Klemmstift 33 gedrückt wird. Durch die hierdurch geschaffene Umlenkung des Bands 23 kann die Klemmkraft auf das Band 23 zusätzlich erhöht werden. Um das Einführen des Bands 23 zu erleichtern, kann auf das einzuführende Ende 21 des Bands 23 eine als Hülse 39 ausgebildete Einführhilfe aufgesteckt sein (Fig. 3a und 3b). Wie in Fig. 3c zu erkennen ist, können ein, zwei, drei oder beliebig viele Klemmstifte 33, 41, 43 vorgesehen sein.

Bei dem Ausführungsbeispiel gemäß Fig. 3d umfasst die Bandbefestigung ein Druckelement, beispielsweise eine Klemmschraube 137, das über eine Öffnung 133 quer zur Einführrichtung 25 in den Hohlraum des Kopfs 13 des Stabs 11 einführbar und zum Klemmen des Endes 21 des Bands 23 innerhalb des Kopfs 13 des Stabs 11 ausgelegt ist. Ferner ist eine gegenüber der Bandeintrittsöffnung 19 tiefer liegende Bandaustrittsöffnung 135 vorgesehen, durch die das Ende 21 des Bands 23 aus dem Hohlraum des Stabs 11 wieder ausführbar oder ausgeführt ist. Der nicht in den Kopf 13 eingeführte Teil des Bands 23 ist von einem Druckkörper 75, der auch als Kissen bezeichnet werden kann, umgeben. Der Stabs 11 ist im zusammengesetzten Zustand in eine Aufnahme 139 einer an ihrem oberen Ende stimmgabelartig ausgebildeten gemeinsamen Pedikelschraube 67 eingelegt oder einlegbar und mittels einer Klemmschraube 141 an dieser befestigt oder befestigbar. Das obere Ende der gemeinsamen Pedikelschraube 67 kann aber auch als Durchgang ausgebildet sein.

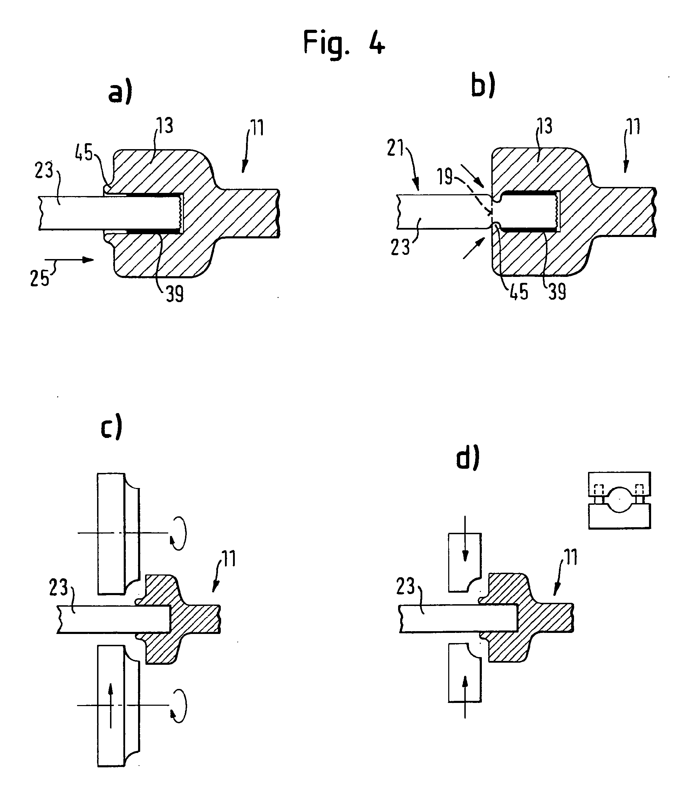

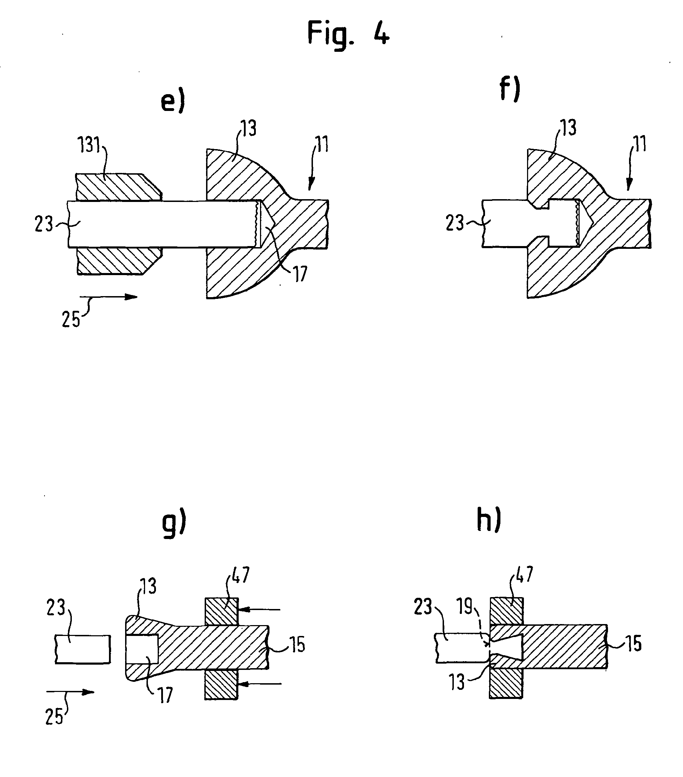

Fig. 4 zeigt ein Stabilisierungssystem gemäß einer vierten Ausführungsform der Erfindung, bei der sich der Hohlraum 17 zur Bandeintrittsöffnung 19 des Kopfes 13 des Stabs 11 hin verengt. Die Verengung ist dabei bei in den Hohlraum 17 eingeführtem Ende 21 des Bands 23 durch Umformen des Kopfes 13 des Stabs 11 entstanden. Durch die Verengung wird das Band 23 wirksam geklemmt (Fig. 4b, 4f und 4h). Analog zu Fig. 3 kann auch bei der Ausführungsform gemäß Fig. 4 eine Hülse 39 als Einführhilfe für das Ende 21 des Bands 23 verwendet werden, wie in den Fig. 4a und 4b dargestellt ist.

In dem Ausführungsbeispiel gemäß den Fig. 4a und 4b weist der Kopf 13 des Stabs 11 vor der Umformung eine entgegen der Einführrichtung 25 vorstehende, in Umfangsrichtung des Stabs 11 umlaufende Auskragung 45 auf. Das Umformen der Auskragung 45 von der in Fig. 4a gezeigten Position in die in Fig. 4b gezeigte Position, bei der die Orientierung der Auskragung 45 gewissermaßen um 90° gedreht wird, kann beispielsweise durch Rollpressen (Fig. 4c) oder durch die Verwendung zweier Halbschalen (Fig. 4d) erreicht werden.

In dem Ausführungsbeispiel gemäß den Fig. 4e und 4f weist der Kopf 13 des Stabs 11 vor der Umformung keine Auskragung auf. Vielmehr wird beim Einpressen des einzuführenden Endes 21 des Bands 23 der die Begrenzung der Bandeintrittsöffnung 19 bildende Bereich des Kopfes 13 des Stabs 11 mittels eines Stempels 131 derart verformt, insbesondere nach innen gedrückt, dass sich der Hohlraum 17 zur Bandeintrittsöffnung 19 des Kopfes 13 des Stabs 11 hin verengt.

Das Ausführungsbeispiel gemäß den Fig. 4g und 4h zeichnet sich dadurch aus, dass vor der Umformung der Außendurchmesser des Kopfes 13 des Stabs 11 und die damit verbundene Wandstärke des Kopfes 13 im Bereich des Hohlraums 17 in die der Einführrichtung 25 des Bands 23 entgegengesetzte Richtung zunimmt. Beim Umformen des Kopfes 13 des Stabs 11 wird ein Pressring 47, der einen Innendurchmesser aufweist, der im Wesentlichen dem Außendurchmesser des Mittelteils 15 des Stabs 11 entspricht, entgegen der Einführrichtung 25 bewegt. Dabei wird der Kopf 13 des Stabs 11 auf den Innendurchmesser des Pressrings 47 zusammengedrückt, so dass sich der Hohlraum 17 zur Bandeintrittsöffnung 19 des Kopfes 13 hin aufgrund der in dieser Richtung zunehmenden Wandstärke des Kopfes 13 verengt.

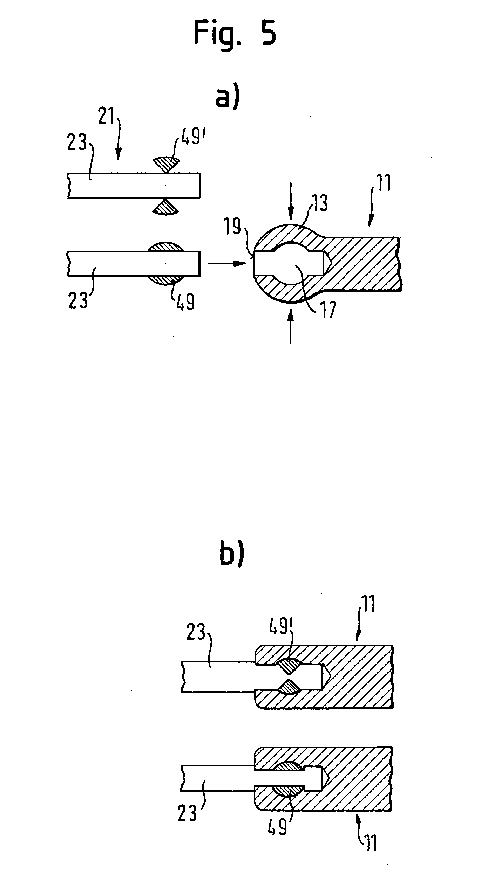

Fig. 5 zeigt ein Stabilisierungssystem gemäß einer fünften Ausführungsform der Erfindung, bei der die Verbindung des Bands 23 mit dem Stab 11 durch Umformen des Kopfes 13 des Stabs 11 entstanden ist. Der Kopf 13 des Stabs 11 sowie der Hohlraum 17 innerhalb des Kopfes 13 sind dabei zumindest vor der Umformung jeweils kugelartig geformt, wobei der Durchmesser der Bandeintrittsöffnung 19 kleiner ist als der in dem Kopf 13 ausgebildete Hohlraum 17. Die Hohlraum 17 ist somit bereits vor der Umformung zur Bandeintrittsöffnung 19 hin verengt.

Zur Herstellung der Verbindung des Bands 23 mit dem Stab 11 wird zunächst ein Ring 49, insbesondere ein zugespitzter Ring 49', auf das Ende 21 des Bands 23 aufgeschoben (Fig. 5a). Das mit dem Ring 49, 49' versehene Ende 21 des Bands 23 wird dann in den Hohlraum 17 des Kopfes 13 des Stabs 11 eingeführt, woraufhin der Kopf 13 des Stabs 11 und somit auch der Ring 49, 49' zusammengepresst wird, so dass das Band 23 wirksam geklemmt ist (Fig. 5b). Durch das Zuspitzen des Rings 49' kann die auf das Band 23 ausgeübte Presskraft zusätzlich erhöht werden.

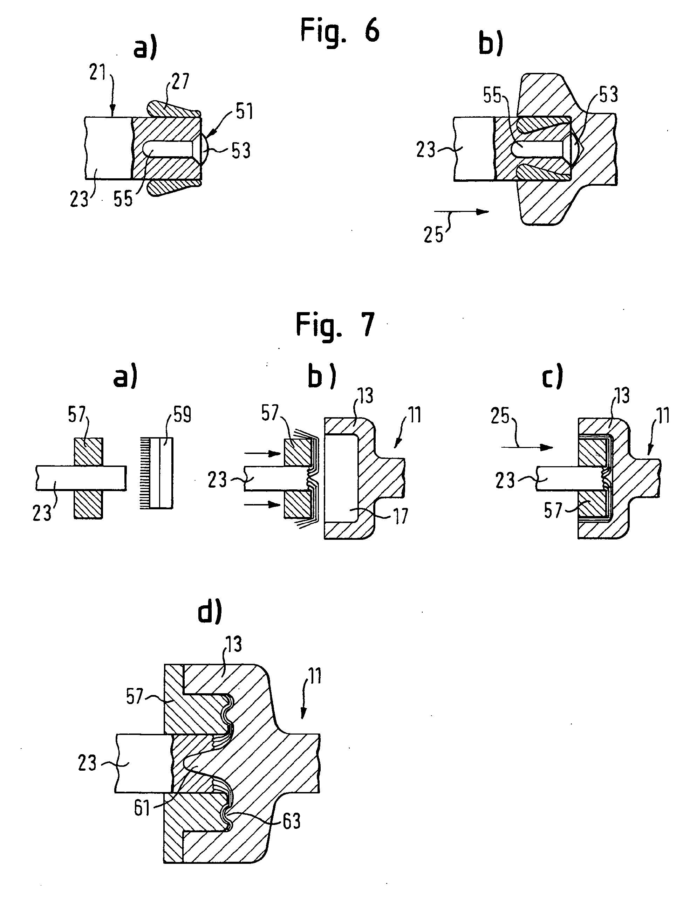

Fig. 6 zeigt ein Stabilisierungssystem gemäß einer sechsten Ausführungsform der Erfindung. Die Ausführungsform gemäß Fig. 6 entspricht im Wesentlichen der Ausführungsform gemäß Fig. 1, mit dem Unterschied, dass die Bandbefestigung zusätzlich eine insbesondere aus Metall oder Kunststoff bestehende Einlage 51 umfasst, die in das in den Hohlraum des Stabs 11 einführbare (Fig. 6a) oder eingeführte (Fig. 6b) Ende 21 des Bands 23 insbesondere zentrisch eingearbeitet ist und sich längs der Einführrichtung 25 des Bands 23 erstreckt.

Die Einlage 51 ist stiftförmig ausgebildet und umfasst einen Kopf 53 und einen Schaft 55, wobei der Durchmesser des Kopfes 53 größer ist als der des Schafts 55. Der Kopf 53 ragt dabei zum Teil aus dem Ende 21 des Bands 23 heraus. Durch die Einlage 51 kann der auf das eingeklemmte Band 23 ausgeübte Pressdruck und somit die Haltekraft der durch die Bandbefestigung ausgebildeten Verbindung zwischen dem Band 23 und dem Stab 11 erhöht werden.

Fig. 7 zeigt ein Stabilisierungssystem gemäß einer siebten Ausführungsform der Erfindung. In Fig. 7 ist das Band 23 zu einer Vielzahl von Festigkeitsträgern, insbesondere Fasern, aus denen das Band 23 besteht, aufgeweitet und mittels eines Einsatzes 57 in den Hohlraum 17 des Kopfs 13 des Stabs 11 einpressbar (Fig. 7b) oder eingepresst (Fig. 7c). Um die Aufweitung zu erreichen, wird das Ende 21 des Bands 23 mittels eines Bürstenwerkzeugs 59 aufgebürstet (Fig. 7a).

Zum Einpressen wird der Einsatz 57, der in Fig. 7 als Ringeinsatz ausgelegt ist, auf das Band 23 aufgefädelt, und in den Hohlraum 17 des Kopfs 13 eingeführt, so dass die einzelnen Fasern, die in allen Richtungen radial zur Einführrichtung 25 abstehen, zwischen der Vorderseite des Ringeinsatzes 57 und der Begrenzung des Hohlraums 17 eingeklemmt sind. Um die Haltekräfte einer derartigen Verbindung zwischen dem Band 23 und dem Stab 11 zu erhöhen, kann ein zentral positionierter Dorn 61, der entgegen der Einführrichtung 25 in den Hohlraum 17 hineinragt, und eine um den Dorn 61 umlaufende Nase 63, die ebenfalls entgegen der Einführrichtung 25 in den Hohlraum 17 hineinragt, vorgesehen sein (Fig. 7d).

Gemäß einer nicht dargestellten achten Ausführungsform der Erfindung ist das in den Hohlraum einführbare oder eingeführte Ende des Bands insbesondere mittels Leim oder Zement verklebt. Hierdurch werden die Fasern des Bandes miteinander verbunden, so dass ein Aneinanderabgleiten der einzelnen Fasern verhindert und somit eine größere Haltekraft für die Verbindung zwischen dem Band und dem Stab erreicht werden kann.

Gemäß einer nicht dargestellten neunten Ausführungsform der Erfindung weist das in den Hohlraum einführbare oder eingeführte Ende des Bands eine Form auf, die durch Schmelzen, Formen und anschließendes Aushärten entstanden ist. Die Form kann insbesondere derart sein, dass das Ende des Bands nach Aushärten als Widerhaken ausgelegt ist.

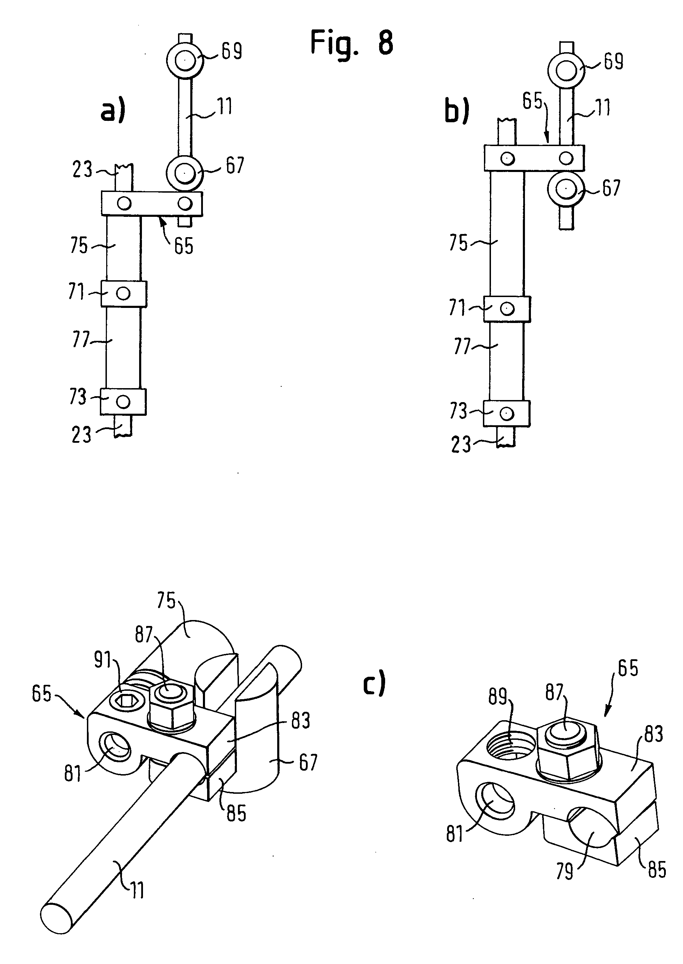

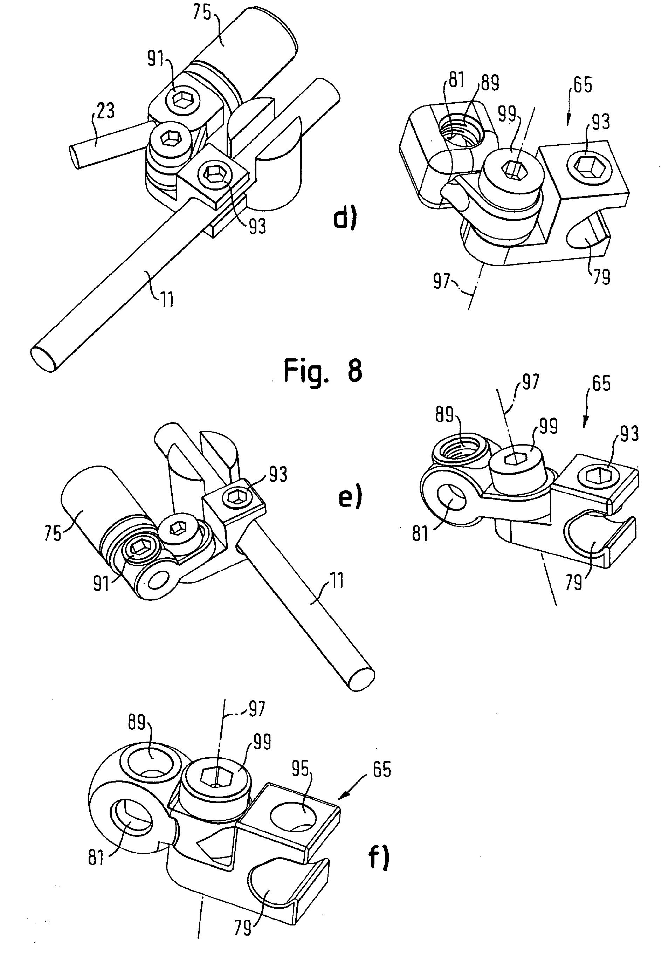

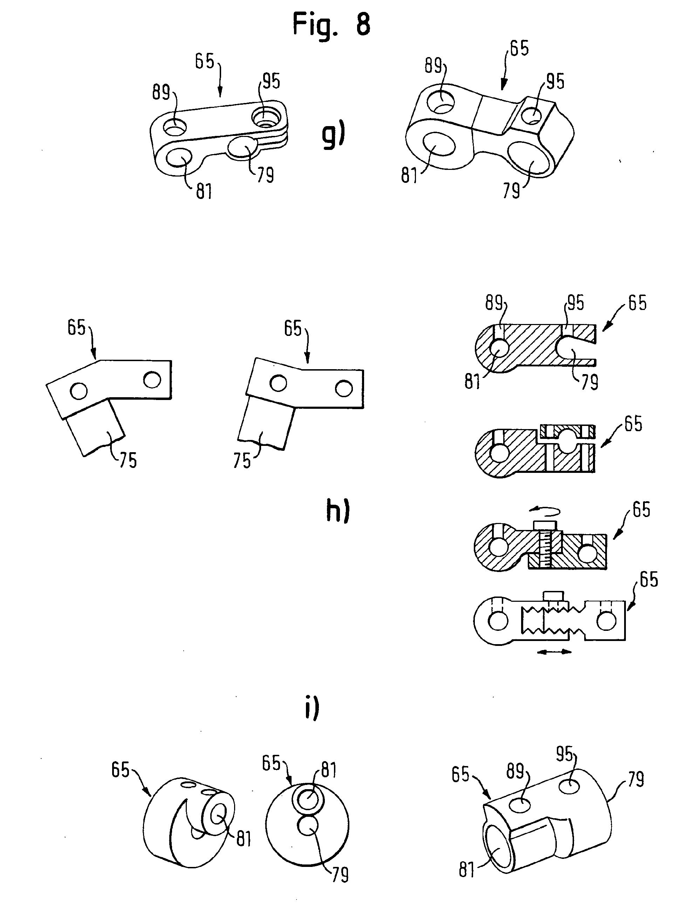

Fig. 8 zeigt ein Stabilisierungssystem gemäß einer zehnten Ausführungsform der Erfindung, bei der die Bandbefestigung eine separate Baueinheit 65 umfasst, wobei der Stab 11 sowohl an der Bandbefestigung, d.h. insbesondere an der separaten Baueinheit 65, als auch an einer gemeinsamen Pedikelschraube 67 befestigbar oder befestigt ist. Insbesondere ist die separate Baueinheit 65 dabei nicht unmittelbar an der gemeinsamen Pedikelschraube 67 befestigbar oder befestigt.

Die Fig. 8a und 8b zeigen jeweils ein Stabilisierungssystem im implantierten Zustand. Der Stab 11 verbindet zwei Pedikelschrauben 67, 69, die die gemeinsame Pedikelschraube 67 umfassen und jeweils an einem Wirbel einer Wirbelsäule befestigt sind, zu einem starren Versteifungssystem. Das Band 23, das auf Zug vorgespannt ist, verbindet drei Pedikelschrauben 67, 71, 73, die die gemeinsame Pedikelschraube 67 umfassen, wobei die Pedikelschrauben 71, 73 ebenfalls jeweils an einem Wirbel befestigt sind, zu einem elastischen Stützsystem. Zwischen den Pedikelschrauben 67 und 71 sowie zwischen den Pedikelschrauben 71 und 73 ist jeweils ein komprimierter Druckkörper 75, 77 angeordnet, die das Band 23 in Umfangsrichtung jeweils vollständig umgeben. Die gemeinsame Pedikelschraube 67 ist sowohl dem Versteifungssystem als auch dem Stützsystem zugeordnet.

Das Band 23 und der Stab 11 sind durch die separate Baueinheit 65 miteinander verbunden. Die separate Baueinheit 65 erstreckt sich im Wesentlichen senkrecht zu der Längserstreckung des Bands 23 bzw. senkrecht zu der Längserstreckung des Stabs 11, die im Wesentlichen parallel und medial/lateral versetzt zueinander verlaufen, wohingegen wenigstens bei den Ausführungsformen gemäß den Figuren 1 bis 7 die Längserstreckung des Bands und die Längserstreckung des Stabs zumindest ohne medialen/lateralen Versatz parallel zueinander verlaufen. Gemäß Fig. 8 kann die separate Baueinheit 65 bezüglich der gemeinsamen Pedikelschraube 67 sowohl auf der Seite des Stützsystems (Fig. 8a) als auch auf der Seite des Versteifungssystems (Fig. 8b) angeordnet sein.

Das Ausführungsbeispiel gemäß Fig. 8c zeigt eine separate Baueinheit 65, die zwei rohrartige Durchgänge 79, 81 zur Befestigung des Bands 23 und zur Befestigung des Stabs 11 aufweist. Der Durchgang 79 zur Befestigung des Stabs 11 weist eine obere und eine untere Halbschale 83, 85 auf, die mittels einer Halteschraube 87 relativ zueinander bewegbar sind, um den Stab 11 einzuklemmen. Senkrecht zu dem Durchgang 81 zur Befestigung des Bands 23 verläuft eine Öffnung 89, in die ein Druckelement 91, beispielsweise eine Klemmschraube, zum Klemmen des Bands 23 an der gemeinsamen Pedikelschraube 67 einsetzbar oder eingesetzt ist.

Die separate Baueinheit 65 kann auch mehrteilig ausgeführt sein, wobei insbesondere unterschiedliche Relativstellungen der Teile der separaten Baueinheit 65 zueinander realisierbar sind. Eine derartige Baueinheit 65 ist jeweils in den Ausführungsbeispielen gemäß den Fig. 8d, 8e und 8f gezeigt. Die separate Baueinheit 65 weist einen rohrartigen Zugang bzw. Durchgang 81 für das Band 23 auf, das mittels der Klemmschraube 91, die durch die Öffnung 89 einführbar ist, befestigbar ist. Der Stab 11, der im implantierten Zustand in den sektorweise offenen Durchgang 79 eingeführt ist, ist analog zu dem Band 23 ebenfalls mittels einer Klemmschraube 93, die durch eine Öffnung 95 einführbar ist, an der separaten Baueinheit 65 befestigbar. Zwischen dem einen Teil der separaten Baueinheit 65, in dem der Zugang bzw. Durchgang 81 ausgebildet ist, und dem anderen Teil der separaten Baueinheit 65, in dem der Durchgang 79 ausgebildet ist, ist eine Drehachse 97 vorgesehen, so dass die Teile der separaten Baueinheit 65 gegeneinander verdrehbar sind. Eine eingestellte Relativstellung der beiden Teile zueinander ist mittels einer Feststellschraube 99 arretierbar.

In den Fig. 8g und 8h sind weitere Ausführungsbeispiele der separaten Baueinheit 65 entweder perspektivisch (Fig. 8g) oder in einer Draufsicht bzw. Querschnittsansicht (Fig. 8h) dargestellt. Insbesondere wird deutlich, dass eine einteilige separate Baueinheit 65 gewinkelt ausgebildet bzw. bei einer mehrteiligen separaten Baueinheit 65 der Abstand der Teile der separaten Baueinheit 65 zueinander durch lineare Verschiebung veränderbar sein kann.

Das Ausführungsbeispiel gemäß 8i zeigt eine separate Baueinheit 65, in die von der einen Seite ein nicht dargestellter Stab und von der anderen, gegenüberliegenden Seite ein nicht dargestelltes Band einführbar ist. Die separate Baueinheit 65, die gewissermaßen als Doppelhülse ausgebildet ist, weist hierfür zwei rohrartige Zugänge 79, 81 auf. Weiterhin sind Öffnungen 89, 95 vorgesehen, um den Stab und das Band mittels nicht dargestellter Klemmschrauben mit der separaten Baueinheit 65 zu verbinden.

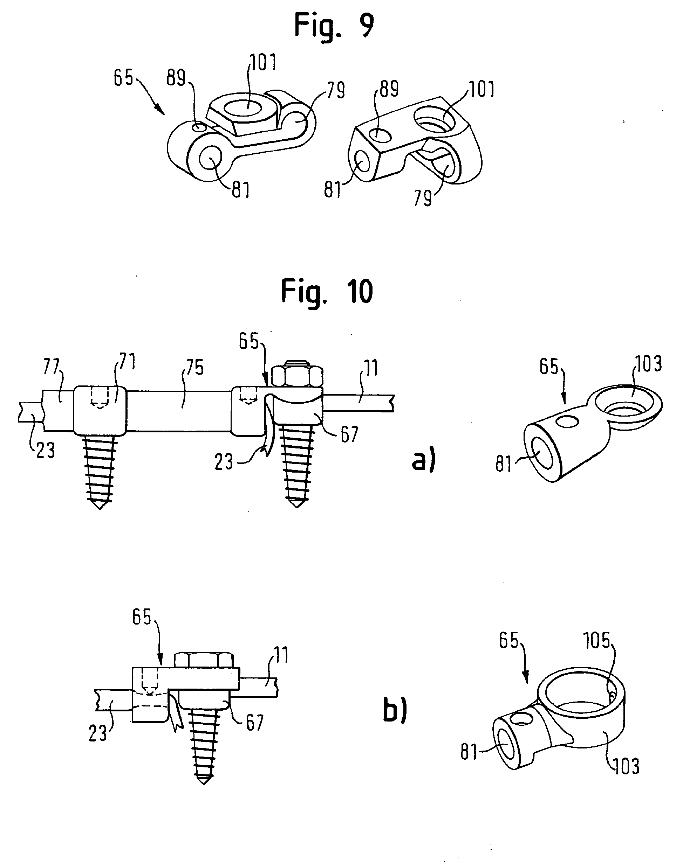

Fig. 9 zeigt ein Stabilisierungssystem gemäß einer elften Ausführungsform der Erfindung, bei der die Bandbefestigung eine separate Baueinheit 65 umfasst, wobei ein nicht dargestellter Stab an der Bandbefestigung 65 und die Bandbefestigung 65 an einer nicht dargestellten gemeinsamen Pedikelschraube befestigbar oder befestigt ist. Insbesondere ist der Stab dabei nicht unmittelbar an der gemeinsamen Pedikelschraube befestigbar oder befestigt.

In dem Ausführungsbeispiel gemäß den Fig. 9a und 9b weist die separate Baueinheit 65 jeweils einen zentralen Durchgang 101 auf, der zur Befestigung der separaten Baueinheit 65 an der gemeinsamen Pedikelschraube vorgesehen ist. Ferner sind in der separaten Baueinheit 65 zwei jeweils senkrecht zu dem zentralen Durchgang 101 und parallel zueinander verlaufende Durchgänge bzw. Zugänge 79, 81 zur Befestigung des Bands und des Stabs ausgebildet. Zur Klemmung des Bands ist eine Öffnung 89 vorgesehen, in die eine nicht dargestellte Klemmschraube einsetzbar ist. Der Durchgang 79 ist geschlitzt und mittels der gemeinsamen Pedikelschraube verengbar, so dass der Stab wirksam einklemmbar ist.

Fig. 10 zeigt ein Stabilisierungssystem gemäß einer zwölften Ausführungsform der Erfindung, bei der die Bandbefestigung eine separate Baueinheit 65 umfasst, wobei die Bandbefestigung und der Stab 11 jeweils an der gemeinsamen Pedikelschraube 67 befestigbar oder befestigt sind. Insbesondere ist der Stab 11 dabei nicht unmittelbar an der separaten Baueinheit 65 befestigbar oder befestigt.

Die separate Baueinheit 65 gemäß Fig. 10a weist eine Hülse bzw. einen Zugang 81 zur Befestigung des Bands 23 und einen Befestigungsring 103 zur Befestigung an der gemeinsamen Pedikelschraube 67 auf, wobei die Längsachse der Hülse 81 und die Mittelachse des Befestigungsrings 103 im Wesentlichen senkrecht aufeinander stehen. Der Befestigungsring 103 kann eine Aussparung 105 für den Stab 11 aufweisen (Fig. 10b).

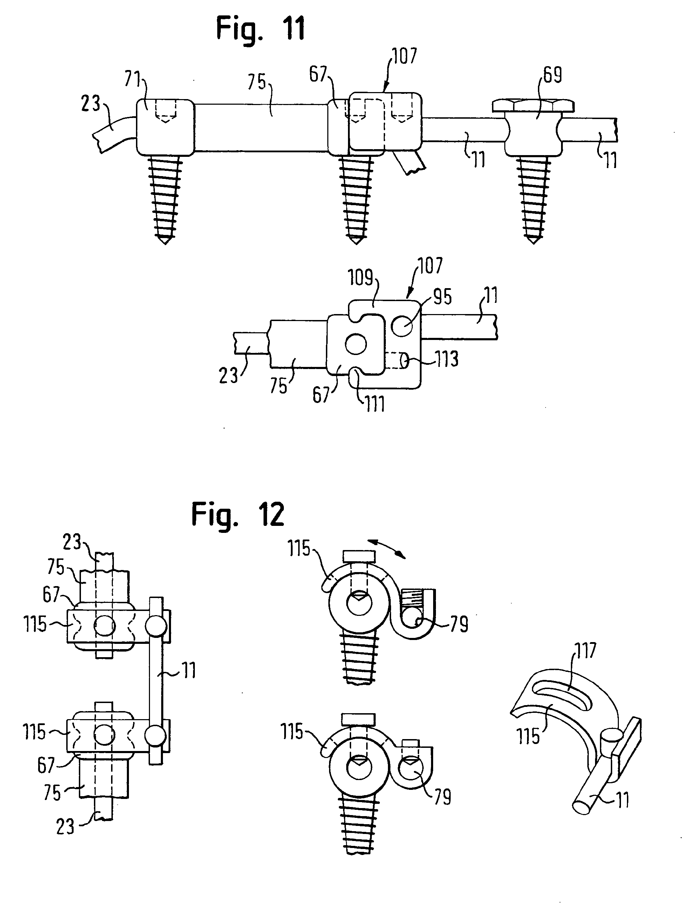

Fig. 11 zeigt ein Stabilisierungssystem gemäß einer dreizehnten Ausführungsform der Erfindung, bei der die Bandbefestigung die gemeinsame Pedikelschraube 67 umfasst, wobei der Stab 11 an einem separaten Bauteil 107 befestigbar oder befestigt ist, das an der gemeinsamen Pedikelschraube 67 befestigbar oder befestigt ist. Insbesondere ist der Stab 11 dabei nicht unmittelbar an der gemeinsamen Pedikelschraube 67 befestigbar oder befestigt.

Das separate Bauteil 107 gemäß Fig. 11 weist zwei seitlich angebrachte Klickarme 109 auf, mit denen das separate Bauteil 107 nach Art eines Klickverschlusses an der gemeinsamen Pedikelschraube 67 befestigbar oder befestigt ist. Zur Befestigung rasten die Klickarme 109 in seitliche Vertiefungen 111 der gemeinsamen Pedikelschraube 67 ein. Darüber hinaus weist das separate Bauteil 107 einen nicht dargestellten Zugang, in den der Stab 11 einführbar oder eingeführt ist, und eine Öffnung 95 auf, in die eine nicht dargestellte Klemmschraube zur Befestigung des Stabs 11 einführbar ist. Darüber hinaus ist eine Öffnung 113 vorgesehen, durch die eine nicht dargestellte Befestigungsschraube zur zusätzlichen Befestigung des separaten Bauteils 107 an der gemeinsamen Pedikelschraube 67 einführbar oder eingeführt ist.

Fig. 12 zeigt ein Stabilisierungssystem gemäß einer vierzehnten Ausführungsform der Erfindung, bei der die Bandbefestigung die gemeinsame Pedikelschraube 67 umfasst, wobei der Stab 11 mittels eines im implantierten Zustand im Wesentlichen senkrecht zur Längserstreckung des Bands 23 verlaufenden Auslegers 115 an der gemeinsamen Pedikelschraube 67 befestigbar oder befestigt ist. Insbesondere ist der Stab 11 dabei nicht unmittelbar an der gemeinsamen Pedikelschraube 67 befestigbar oder befestigt.

Das in Fig. 12 dargestellte Stabilisierungssystem zeigt ein Versteifungssystem, an dessen beiden Enden jeweils ein Stützsystem anschließt, d.h. es sind zwei gemeinsame Pedikelschrauben 67 und zwei Ausleger 115 vorhanden. Die im Wesentlichen eine S-förmige Gestalt aufweisenden Ausleger 115 können jeweils an ihrem dem Stab 11 zugeordneten Ende einen sektorweise offenen Durchgang 79 oder einen in Umfangsrichtung geschlossenen Durchgang 79 aufweisen. Zur Befestigung eines Auslegers 115 an einer gemeinsamen Pedikelschraube 67 ist an dem der gemeinsamen Pedikelschraube 67 zugeordneten Ende ein Langloch 117 ausgebildet, so dass verschiedene Relativstellungen zwischen der gemeinsamen Pedikelschraube 67 und dem Stab 11 möglich sind. Die Längserstreckung des Stabs 11 und die Längserstreckung des Bands 23 verlaufen parallel und medial/lateral versetzt zueinander.

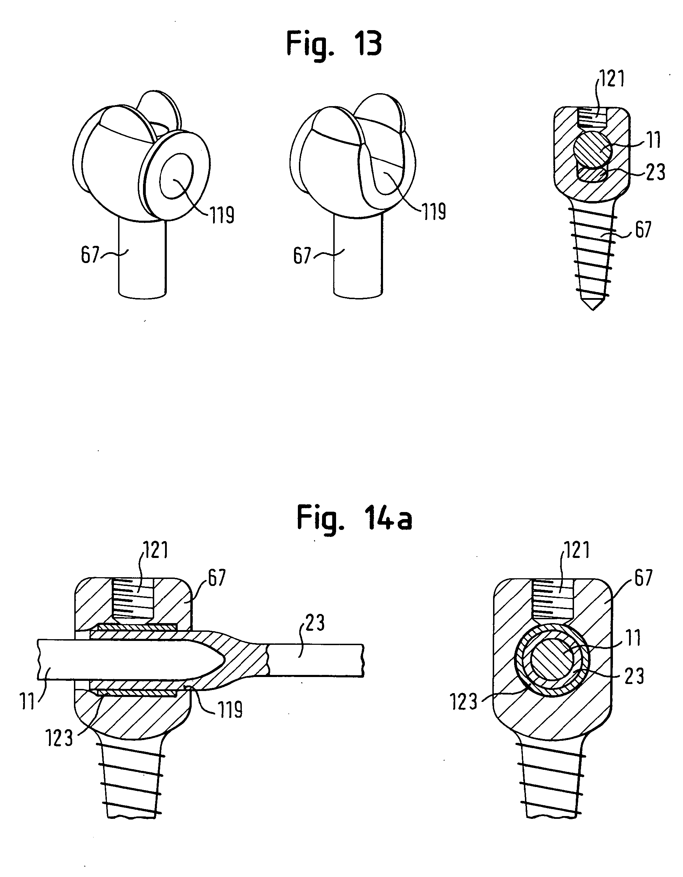

Fig. 13 zeigt ein Stabilisierungssystem gemäß einer fünfzehnten Ausführungsform der Erfindung, bei der die Bandbefestigung die gemeinsame Pedikelschraube 67 umfasst, wobei der Stab 11 an der gemeinsamen Pedikelschraube 67 befestigbar oder befestigt ist und die gemeinsame Pedikelschraube 67 einen gemeinsamen Durchgang 119 für das Band 23 und den Stab 11 aufweist.

In dem Ausführungsbeispiel gemäß Fig. 13 ist das Band 23 und der Stab 11 nebeneinander bzw. übereinander innerhalb des gemeinsamen Durchgangs 119 der gemeinsamen Pedikelschraube 67 angeordnet. Das Band 23 und der Stab 11 werden gemeinsam mittels einer nicht dargestellten Klemmschraube, die durch eine in der gemeinsamen Pedikelschraube 67 ausgebildete Öffnung 121 einführbar oder eingeführt ist, zusammengedrückt, so dass sowohl das Band 23 als auch der Stab 11 wirksam geklemmt werden.

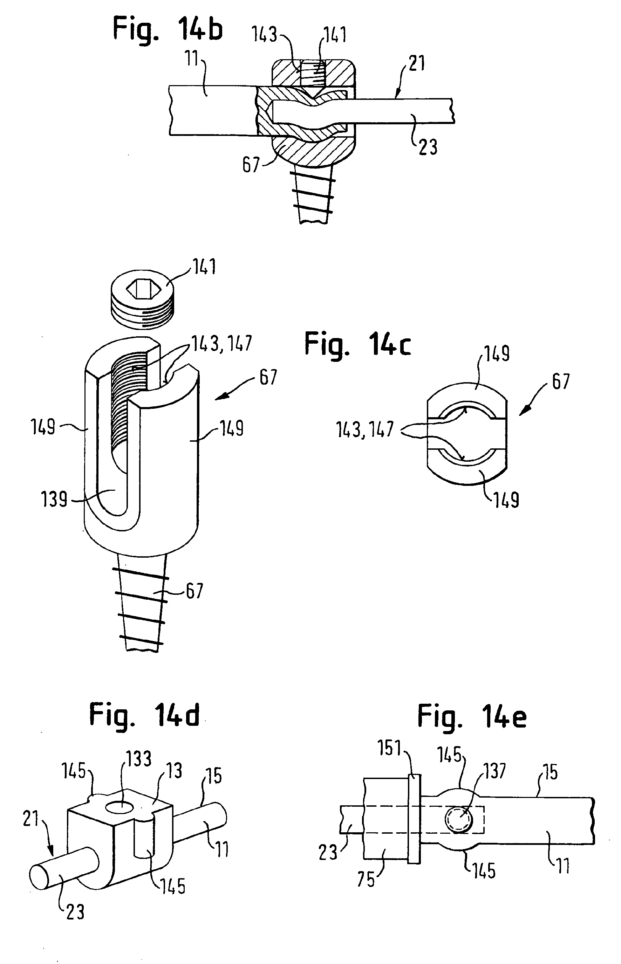

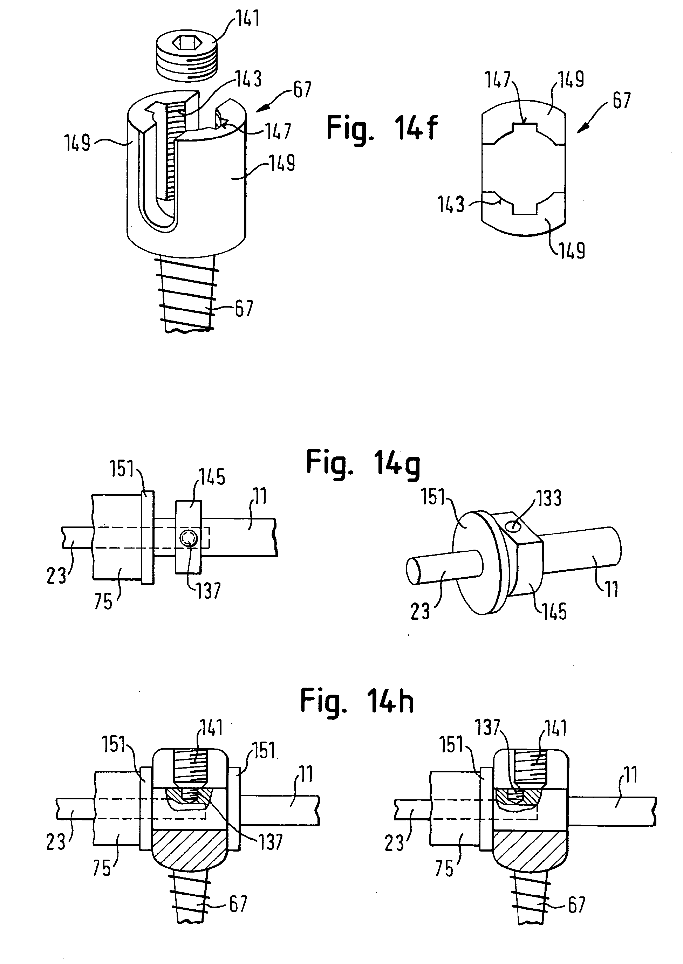

Fig. 14 zeigt ein Stabilisierungssystem gemäß einer sechzehnten Ausführungsform der Erfindung. Die Ausführungsform gemäß Fig. 14 weist analog zu der Ausführungsform gemäß Fig. 13 einen gemeinsamen Durchgang 119 für das Band 23 und den Stab 11 sowie eine Öffnung 121 zum Einführen einer Klemmschraube auf.

Bei dem Ausführungsbeispiel gemäß Fig. 14a ist im Gegensatz zu der Ausführungsform gemäß Fig. 13 das Band 23 über den Stab 11 stülpbar oder gestülpt. Darüber hinaus kann in den gemeinsamen Durchgang 119 eine Hülse 123 eingeschoben oder einschiebbar sein, die ihrerseits wiederum das Band 23 umgibt. Ansonsten sind das Band 23 und der Stab 11 analog zu der Ausführungsform in Fig. 13 einklemmbar. In Fig. 14b ist ein Ausführungsbeispiel gezeigt, bei dem das Ende 21 des Bands 23 ähnlich wie in den Fig. 1 bis 7 in den Stab 11 einführbar oder eingeführt ist, wobei mittels einer Klemmschraube 141, die durch eine Öffnung 143 in die gemeinsame Pedikelschraube 67 einführbar oder eingeführt ist, das Band 23 und der Stab 11 einklemmbar sind.

Bei dem Ausführungsbeispiel gemäß Fig. 14c ist die gemeinsame Pedikelschraube 67 an ihrem oberen Ende stimmgabelartig ausgebildet, so dass zwischen zwei Zinken 149 eine Aufnahme 139 ausgebildet ist, in die ein Stab einlegbar oder eingelegt ist. Die Halterung des Stabs an der gemeinsamen Pedikelschraube 67 erfolgt mittels einer Klemmschraube 141, die in Bohrabschnitte 143 der beiden Zinken 149 einsetzbar oder eingesetzt ist, wobei die Bohrabschnitte 143 jeweils ein Innengewinde aufweisen. Das Ende 13 des Stabs 11, das zur Verbindung mit dem Ende 21 des Bands 23 ausgelegt ist, kann dabei einen Durchmesser aufweisen, der größer ist als der eines Mittelteils 15 des Stabs 11 (Fig. 14d). Das Band 23 ist mittels einer Klemmschraube 137, die durch eine Öffnung 133 in den Stab 11 einführbar oder eingeführt ist, mit dem Stab 11 verbindbar oder verbunden.

Zur Führung des Stabs 11 beim Zusammensetzen mit der gemeinsamen Pedikelschraube 67 bzw. zur korrekten Positionierung des Stabs 11 bezüglich der gemeinsamen Pedikelschraube 67 sind am Stab 11 oder am Kopf 13 des Stabs 11 Führungsmittel, beispielsweise ein oder mehrere runde oder eckige Zapfen 145, ausgebildet (Fig. 14d, 14e, 14g), die in entsprechende Führungsmittel der gemeinsamen Pedikelschraube 67 (Fig. 14c, 14f), beispielsweise eine oder mehrere Ausnehmungen, Rillen oder Nuten 147, die insbesondere den Bohrabschnitten 143 der beiden Zinken 149 entsprechen (Fig. 14c), eingreifen.

Darüber hinaus kann zumindest ein senkrecht zu der Längsachse des Stabs 11 orientierter, insbesondere tellerförmiger Flansch 151 vorgesehen sein (Fig. 14e, 14g, 14h), der mit dem Stab 11 verbunden ist und eine korrekte Positionierung des Stabs 11 bezüglich der gemeinsamen Pedikelschraube 67 erlaubt bzw. eine Führung beim Zusammensetzen des Stabilisierungssystems bietet. Ist der Flansch 151 als Abschluss des Endes 13 des Stabs 11 ausgelegt, kann der Flansch gleichzeitig als Anschlag 151 für einen Druckkörper 75 eines Stützsystems des Stabilisierungssystems dienen.

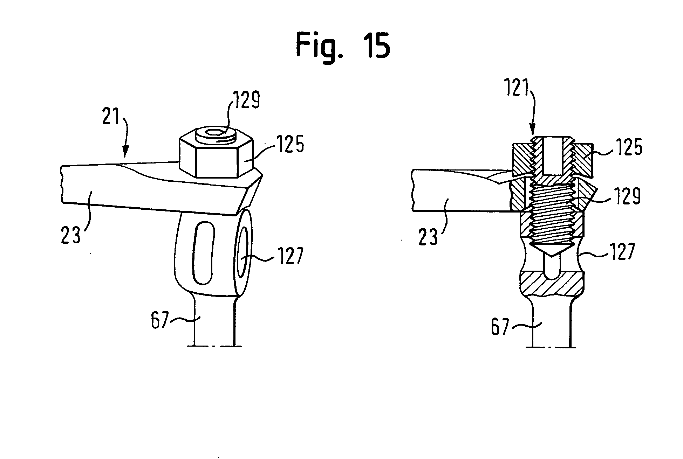

Fig. 15 zeigt ein Stabilisierungssystem gemäß einer siebzehnten Ausführungsform der Erfindung, bei der die Bandbefestigung die gemeinsame Pedikelschraube 67 umfasst, wobei ein nicht dargestellter Stab an der gemeinsamen Pedikelschraube 67 befestigbar oder befestigt ist und das Band 23 zwischen einem Druckorgan, insbesondere einer Schraubenmutter 125, und der gemeinsamen Pedikelschraube 67 einklemmbar oder eingeklemmt ist, wobei die gemeinsame Pedikelschraube 67 einen Durchgang 127 für den Stab aufweist.

Der in den Durchgang 127 eingeführte Stab wird mittels eines Gewindestifts 129, der ein durchgehendes Außengewinde aufweist und durch eine Öffnung 121 in die gemeinsame Pedikelschraube 67 einführbar oder eingeführt ist, wirksam geklemmt. Auf das aus der gemeinsamen Pedikelschraube 67 hervorstehende Ende des Gewindestifts 129 wird die Schraubenmutter 125 aufgesetzt, um das Band 23 zwischen der gemeinsamen Pedikelschraube 67 und der Schraubenmutter 125 einzuklemmen. Das einzuklemmende Ende 21 des Bands 23 ist hierfür als Schlaufe ausgeführt, in die der Gewindestift 129 eingreift.

### Bezugszeichenliste

- 11: Stab
- 13: Kopf
- 15: Mittelteil
- 17: Hohlraum
- 19: Bandeintrittsöffnung
- 21: Ende
- 23: Band
- 25: Einführrichtung
- 27: Klemmhülse
- 29: Vorsprung
- 31: Klemmhülse
- 33: Klemmstift
- 35: Öffnung
- 37: Mulde
- 39: Einführhilfe
- 41: Klemmstift
- 43: Klemmstift
- 45: Auskragung
- 47: Pressring
- 49, 49': Ring, zugespitzter Ring
- 51: Einlage
- 53: Kopf
- 55: Schaft
- 57: Einsatz
- 59: Bürstenwerkzeug
- 61: Dorn
- 63: Nase
- 65: separate Baueinheit
- 67: gemeinsamen Pedikelschraube
- 69: Pedikelschraube
- 71: Pedikelschraube
- 73: Pedikelschraube
- 75: Druckkörper
- 77: Druckkörper
- 79: Durchgang, Zugang
- 81: Durchgang, Zugang, Hülse
- 83: obere Halbschale
- 85: untere Halbschale
- 87: Halteschraube
- 89: Öffnung
- 91: Klemmschraube
- 93: Klemmschraube
- 95: Öffnung
- 97: Drehachse
- 99: Feststellschraube
- 101: zentraler Durchgang
- 103: Befestigungsring
- 105: Aussparung
- 107: separates Bauteil
- 109: Klickarm
- 111: Vertiefung
- 113: Öffnung
- 115: Ausleger
- 117: Langloch
- 119: gemeinsamer Durchgang
- 121: Öffnung
- 123: Hülse
- 125: Schraubenmutter
- 127: Durchgang
- 129: Gewindestift
- 131: Stempel
- 133: Öffnung
- 135: Bandaustrittsöffnung
- 137: Klemmschraube
- 139: Aufnahme
- 141: Klemmschraube
- 143: Öffnung, Bohrabschnitte
- 145: Zapfen
- 147: Ausnehmung
- 149: Zinken
- 151: Flansch, Anschlag

## Patentansprüche

1. Intervertebrales Stabilisierungssystem für wenigstens drei Wirbel, mit
- an den Wirbeln befestigbaren Pedikelschrauben (67, 69, 71, 73),
- einem Stab (11) zum Verbinden von zumindest zwei Pedikelschrauben (67, 69) zu einem starren Versteifungssystem, und
- einem auf Zug vorspannbaren Band (23), das im implantierten Zustand des Stabilisierungssystems von wenigstens einem zwischen zwei benachbarten Pedikelschrauben (67, 71, 73) angeordneten komprimierbaren Druckkörper (75, 77) umgeben ist, zum Verbinden der Pedikelschrauben (67, 71, 73) zu einem elastischen Stützsystem,
wobei eine gemeinsame Pedikelschraube (67) sowohl dem Versteifungssystem als auch dem Stützsystem zugeordnet und das Band (23) mittels einer Bandbefestigung mit dem Stab (11) verbindbar oder verbunden ist.

2. Stabilisierungssystem nach Anspruch 1,
**dadurch gekennzeichnet ,**
**dass** die Bandbefestigung als Klemmeinrichtung zur Klemmung des Bands (23) ausgebildet ist.

3. Stabilisierungssystem nach Anspruch 1 oder 2,
**dadurch gekennzeichnet ,**
**dass** die Bandbefestigung ein Ende (13) des Stabs (11) umfasst, wobei bevorzugt der Stab (11) mit der gemeinsamen Pedikelschraube (67) verbindbar ist.

4. Stabilisierungssystem nach Anspruch 3,
**dadurch gekennzeichnet,**
**dass** das Ende (13) des Stabs (11) einen größeren Durchmesser aufweist als zumindest das Mittelteil (15) des Stabs (11).

5. Stabilisierungssystem nach Anspruch 3 oder 4,
**dadurch gekennzeichnet ,**
**dass** das Ende (13) des Stabs (11) einen Hohlraum (17) aufweist, in den über eine Öffnung (19) ein Ende (21) des Bands (23) einführbar oder eingeführt ist.

6. Stabilisierungssystem nach Anspruch 5,
**dadurch gekennzeichnet ,**
**dass** die Bandbefestigung zumindest ein in den Hohlraum (17) längs der Einführrichtung (25) des Endes (21) des Bands (23) einführbares oder eingeführtes, insbesondere ring- oder hülsenförmiges Klemmelement (27, 31 ) umfasst, das zur Klemmung des in den Hohlraum (17) des Stabs (11) eingeführten Endes (21) des Bands (23) mit dem Ende (13) des Stabs (11) ausgelegt ist.

7. Stabilisierungssystem nach Anspruch 5 oder 6,
**dadurch gekennzeichnet ,**
**dass** die Bandbefestigung zumindest ein insbesondere unter Press-Sitz in den Hohlraum (17) quer zur Einführrichtung (25) des Endes (21) des Bands (23) einführbares, insbesondere stiftförmiges Klemmelement (33, 41, 43) umfasst, das zur Klemmung eines in den Hohlraum (17) des Stabs (11) eingeführten Endes (21) des Bands (23) mit dem Ende (13) des Stabs (11) ausgelegt ist.

8. Stabilisierungssystem nach einem der Ansprüche 5 bis 7,
**dadurch gekennzeichnet ,**
**dass** sich der Hohlraum (17) zur Öffnung (19) hin verengt.

9. Stabilisierungssystem nach Anspruch 8,
**dadurch gekennzeichnet ,**
**dass** die Verengung bei in den Hohlraum (17) eingeführtem Ende (21) des Bands (23) durch Umformen oder Pressen des Endes (13) des Stabs (11) entstanden ist.

10. Stabilisierungssystem nach einem der Ansprüche 5 bis 9,
**dadurch gekennzeichnet ,**
**dass** die Bandbefestigung zusätzlich eine insbesondere aus Metall oder Kunststoff bestehende Einlage (51) umfasst, die in das in den Hohlraum (17) des Stabs (11) einführbare oder eingeführte Ende (21) des Bands (23) eingearbeitet ist.

11. Stabilisierungssystem nach einem der Ansprüche 5 bis 10,
**dadurch gekennzeichnet ,**
**dass** das Ende (21) des Bands (23) zu einer Vielzahl von Festigkeitsträgern des Bands (23) aufgeweitet, insbesondere aufgebürstet, und mittels eines Einsatzes (57) in den Hohlraum (17) des Stabs (11) einpressbar oder eingepresst ist.

12. Stabilisierungssystem nach einem der Ansprüche 5 bis 11,
**dadurch gekennzeichnet ,**
**dass** das in den Hohlraum (17) des Stabs (11) einführbare oder eingeführte Ende (21) des Bands (23) verklebt ist.

13. Stabilisierungssystem nach einem der Ansprüche 5 bis 12,
**dadurch gekennzeichnet ,**
**dass** das in den Hohlraum (17) des Stabs (11) einführbare oder eingeführte Ende (21) des Bands (23) eine Form aufweist, die durch Schmelzen, Formen und anschließendes Aushärten entstanden ist.

14. Stabilisierungssystem nach einem der vorhergehenden Ansprüche,
**dadurch gekennzeichnet ,**
**dass** die Bandbefestigung eine separate Baueinheit (65) umfasst.

15. Stabilisierungssystem nach Anspruch 14,
**dadurch gekennzeichnet ,**
**dass** die Bandbefestigung (65) zwei insbesondere rohrartige Zugänge oder Durchgänge (79, 81) zur Befestigung des Bands (23) und zur Befestigung des Stabs (11) aufweist.

16. Stabilisierungssystem nach Anspruch 14 oder 15,
**dadurch gekennzeichnet ,**
**dass** die Bandbefestigung (65) mehrteilig ausgebildet ist, wobei insbesondere unterschiedliche Relativstellungen der Teile der Bandbefestigung (65) zueinander realisierbar sind.

17. Stabilisierungssystem nach einem der Ansprüche 14 bis 16,
**dadurch gekennzeichnet ,**
**dass** der Stab (11) sowohl an der Bandbefestigung (65) als auch an der gemeinsamen Pedikelschraube (67) befestigbar oder befestigt ist.

18. Stabilisierungssystem nach einem der Ansprüche 14 bis 16,
**dadurch gekennzeichnet ,**
**dass** der Stab (11) an der Bandbefestigung (65) und die Bandbefestigung (65) an der gemeinsamen Pedikelschraube (67) befestigbar oder befestigt ist.

19. Stabilisierungssystem nach einem der Ansprüche 14 bis 16,
**dadurch gekennzeichnet ,**
**dass** die Bandbefestigung (65) und der Stab (11) jeweils an der gemeinsamen Pedikelschraube (67) befestigbar oder befestigt sind.

20. Stabilisierungssystem nach Anspruch 19,
**dadurch gekennzeichnet ,**
**dass** die Bandbefestigung (69) eine Hülse (81) zur Befestigung des Bands (23) und einen Ring (103) zur Befestigung an der gemeinsamen Pedikelschraube (67) aufweist, wobei die Längsachse der Hülse (81) und die Mittelachse des Rings (103) im Wesentlichen senkrecht aufeinander stehen.

21. Stabilisierungssystem nach einem der vorhergehenden Ansprüche,
**dadurch gekennzeichnet ,**
**dass** die Bandbefestigung die gemeinsame Pedikelschraube (67) umfasst.

22. Stabilisierungssystem nach Anspruch 21,
**dadurch gekennzeichnet ,**
**dass** der Stab (11) an einem separaten Bauteil (107, 115) befestigbar oder befestigt ist, das an der gemeinsamen Pedikelschraube (67) befestigbar oder befestigt ist.

23. Stabilisierungssystem nach Anspruch 22,
**dadurch gekennzeichnet ,**
**dass** das separate Bauteil (107) nach Art eines Klick-, Clips-, Rast- oder Schnappverschlusses an der gemeinsamen Pedikelschraube (67) befestigbar oder befestigt ist.

24. Stabilisierungssystem nach Anspruch 22,
**dadurch gekennzeichnet ,**
**dass** der Stab (11) mittels eines im implantierten Zustand im Wesentlichen senkrecht zu der Längserstreckung des Bands (23) verlaufenden Auslegers (115) an der gemeinsamen Pedikelschraube (67) befestigbar oder befestigt ist.

25. Stabilisierungssystem nach Anspruch 21,
**dadurch gekennzeichnet ,**
**dass** der Stab (11) an der gemeinsamen Pedikelschraube (67) befestigbar oder befestigt ist.

26. Stabilisierungssystem nach Anspruch 25,
**dadurch gekennzeichnet ,**
**dass** die gemeinsame Pedikelschraube (67) einen gemeinsamen Durchgang (119) für das Band (23) und den Stab (11) aufweist, wobei insbesondere das Band (23) über den Stab (11) stülpbar oder gestülpt ist.

27. Stabilisierungssystem nach Anspruch 25,
**dadurch gekennzeichnet ,**
**dass** das Band (23) zwischen einem Druckorgan (125) und der gemeinsamen Pedikelschraube (67) einklemmbar oder eingeklemmt ist, wobei die gemeinsame Pedikelschraube (67) einen Durchgang (127) für den Stab (11) aufweist.

28. Stabilisierungssystem nach einem der Ansprüche 1 bis 27,
**dadurch gekennzeichnet ,**
**dass** zumindest ein Druckelement (91, 93), beispielsweise eine Schraube, zum Klemmen des Bands (23) und/oder des Stabs (11) an der gemeinsamen Pedikelschraube (67) vorgesehen ist.

29. Stabilisierungssystem nach einem der Ansprüche 1 bis 28,
**dadurch gekennzeichnet ,**
**dass** im implantierten Zustand die Längserstreckung des Versteifungssystems und die Längserstreckung des Stützsystems parallel und medial/lateral versetzt zueinander verlaufen.

30. Stabilisierungssystem nach einem der Ansprüche 1 bis 28,
**dadurch gekennzeichnet ,**
**dass** im implantierten Zustand die Längserstreckung des Versteifungssystems und die Längserstreckung des Stützsystems zumindest ohne medialen/lateralen Versatz parallel zueinander verlaufen.
